Europäisches Patentamt

European Patent Office

Office européen des brevets

(19)

(11) Veröffentlichungsnummer : **0 144 501**
**B1**

(12) **EUROPÄISCHE PATENTSCHRIFT**

(45) Veröffentlichungstag der Patentschrift :
05.04.89

(21) Anmeldenummer : 84107178.0

(22) Anmeldetag : 22.06.84

(51) Int. Cl.⁴ : **A 61 M 16/20**

(54) Steuerbares Membranventil für Beatmungsgeräte.

(30) Priorität : 18.11.83 DE 3341711

(43) Veröffentlichungstag der Anmeldung :
19.06.85 Patentblatt 85/25

(45) Bekanntmachung des Hinweises auf die Patenterteilung : 05.04.89 Patentblatt 89/14

(84) Benannte Vertragsstaaten :
FR GB IT

(56) Entgegenhaltungen :
AT–B– 237 449
AU–B– 4 486 679
DE–B– 1 491 671
DE–B– 1 491 740
DE–B– 2 331 525
DE–C– 2 947 363
Die Akte enthält technische Angaben, die nach dem
Eingang der Anmeldung eingereicht wurden und die
nicht in dieser Patentschrift enthalten sind.

(73) Patentinhaber : **Drägerwerk Aktiengesellschaft**
**Moislinger Allee 53-55**
**D-2400 Lübeck 1 (DE)**

(72) Erfinder : **Slemmer, Janos, Dr.**
**Georgstrasse 41**
**D-2400 Lübeck (DE)**
Erfinder : **Schröter, Gottfried**
**Hüxtertorallee 55a**
**D-2400 Lübeck (DE)**

EP 0 144 501 B1

## Beschreibung

Die Erfindung betrifft ein steuerbares Membranventil für Beatmungsgeräte mit einer Gaszufuhrleitung, die im Ventilgehäuse in eine im wesentlichen zylindrische Ventilkammer führt, dort in einem ringförmigen Ventilsitz endet, sowie mit einer aus der Kammer herausführenden Gasauslaßleitung und einer die Ventilkammer überspannenden Membran, mit welcher der Ventilsitz verschließbar ist und die abgetrennt von der Ventilkammer eine Steuerkammer ausbildet, die einen Gaszuführstutzen und einen Gasabführstutzen aufweist.

Ein derartiges steuerbares Membranventil ist bereits aus dem US-Patent 38 26 255 bekannt.

Im allgemeinen wird ein Membranventil nach der Erfindung in dem Gaskreislauf eines maschinellen Beatmungssystems verwendet. Es enthält in einem Ventilgehäuse eine Ventilkammer, durch die die beim Ausatmen über eine Gaszuführ- und -auslaßleitung vom Patienten ausgeatmeten Gase hindurchgeleitet werden. Beim Einatmen jedoch verschließt die Membran die Gaszuführleitung und verhindert dadurch das Abfließen des einzuatmenden Gases. Der Verschluß wird durch einen Überdruck in der Steuerkammer oberhalb der Membran erreicht.

Aus der US-PS 38 26 255 ist ein Ausatemventil bekannt, das eine im wesentlichen zylindrische Ventilkammer aufweist, in die eine in die Kammer führende Gaszuführleitung, die in einem ringförmigen Ventilsitz endet, hineinführt und eine Gasauslaßleitung besitzt. Die Kammer wird von einer Membran überspannt, mit welcher der Ventilsitz verschließbar ist. Sie bildet in dem Ventilgehäuse abtrennend von der Ventilkammer eine Steuerkammer. Die Membran wird von einem Deckel gehalten, der dann auch die Steuerkammer ausbildet. Gaszuführungen im Deckel verbinden die Steuerkammer mit dem Steuerdruck. In der bekannten DE-PS 29 47 363 wird das oben genannte Ausatemventil benutzt. Es besitzt in der Mitte des Deckels einen Gaseinlaßstutzen, der dazu verwendet werden kann, ein Gas in die Steuerkammer einzuführen, mit dem der Druck oberhalb der Membran dann reguliert werden kann.

Diese bekannten Membranventile verlangen zur Steuerung der Membran eine besondere Steuergaseinrichtung, die dann ein weiteres, zusätzlich risikoerhöhendes Bauteil darstellt.

Aufgabe der Erfindung ist ein druckgesteuertes Membranventil für Beatmungsgeräte, das eingesetzt in das Beatmungssystem direkt gesteuert wird.

Diese Aufgabe wird erfindungsgemäß dadurch gelöst, daß der Gasabführstutzen eine Blende enthält.

Die mit der Erfindung erzielten Vorteile bestehen insbesondere darin, daß der Fluß des Atemgases innerhalb des Beatmungsgerätes dazu benutzt wird, einen sicheren Schließdruck für das Membranventil, also das Auflegen der Membran auf den Ventilsitz der Gaszuführleitung, zu erreichen. Durch die Blende in dem Gasabführstutzen aus der Steuerkammer heraus kommt es hier durch die Drosselwirkung immer zu einem Staudruck, der zu einer positiven Druckdifferenz, also einer Druckerhöhung in der Steuerkammer, führt.

Das Öffnungsverhältnis Gasabführstutzen-Innendurchmesser : Blenden-Lochdurchmesser 2 : 1 gibt für besonders empfindliche Steuerungen den günstigsten Steuerkammerdruck.

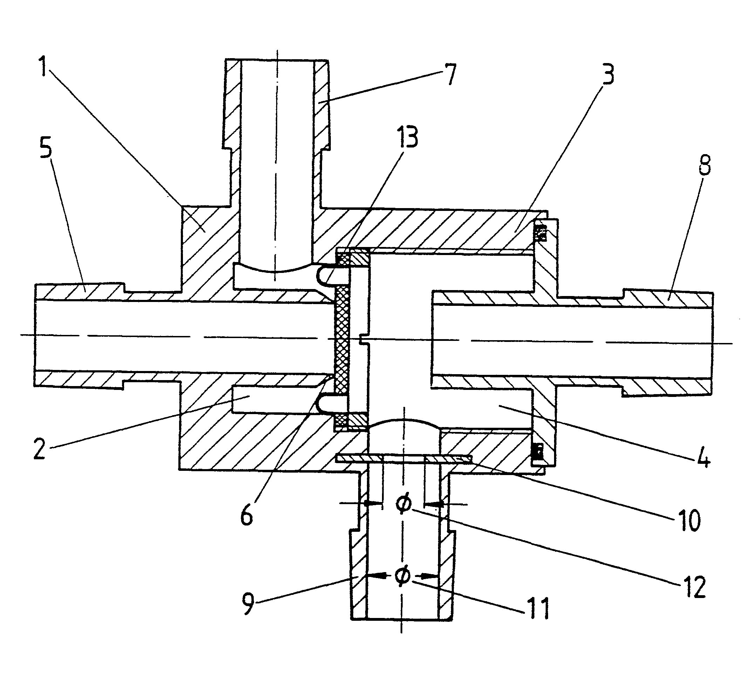

Ein Ausführungsbeispiel der Erfindung ist in der Zeichnung dargestellt und wird im folgenden beschrieben.

Das steuerbare Membranventil weist ein im wesentlichen zylindrisches Ventilgehäuse auf, das im Ventilteil 1 eine Ventilkammer 2 und im Steuerteil 3 eine Steuerkammer 4 bildet.

Der Ventilteil 1 besitzt eine Gaszuführleitung 5, die in der Ventilkammer 2 in einem ringförmigen Ventilsitz 6 endet, und eine Gasauslaßleitung 7. Der Steuerteil 3 trägt einen Gaszuführstutzen 8 sowie einen Gasabführstutzen 9, der eine Blende 10 enthält. Das Öffnungsverhältnis des GasabführstutzenInnendurchmessers 11 zum Blenden-Lochdurchmesser 12 beträgt 2 : 1.

Die Ventilkammer 2 ist durch eine Membran 13 von der Steuerkammer 4 abgetrennt. Die Membran 13 verschließt über eine positive Druckdifferenz in der Steuerkammer 4 zur Ventilkammer 2 am Ventilsitz 6 die Gaszuführleitung 5. Die Schaltstellungen des Membranventils ergeben sich als :

a) geschlossen b) geöffnet.

Zu a) Das Einatemgas fließt über den Gaszuführstutzen 8, durch die Steuerkammer 4, über die Blende 10 und den Gasabführstutzen 9 zum Patienten. Die Ausatemleitung ist in der Gaszuführleitung 5 mit der Membran 13 am Ventilsitz 6 verschlossen. Der Schließdruck für die Membran 13 resultiert aus der positiven Druckdifferenz in der Steuerkammer 4 zur Ventilkammer 2, die mit der Strömung des Atemgases durch die Blende 10 infolge der Drosselwirkung entsteht.

Zu b) Mit der Ansaugung des Atemgases vom Patienten über die Steuerkammer 4 entsteht in dieser ebenfalls über die Drosselwirkung an der Blende 10 eine negative Druckdifferenz. Die Membran 13 hebt vom Ventilsitz 6 ab. Die Ausatemleitung vom Patienten über die Gaszuführleitung 5, durch die Ventilkammer 2 und die Gasauslaßleitung 7 ist geöffnet.

## Patentansprüche

1. Steuerbares Membranventil für Beatmungsgeräte mit einer Gaszuführleitung (5), die im Ventilgehäuse (1) in eine im wesentlichen zylindrische Ventilkammer (2) führt, dort in einem ringförmigen Ventilsitz (6) endet, sowie mit einer aus der Ventilkammer (2) herausführenden Gasauslaßleitung (7) und einer die Ventilkammer (2) überspannenden Membran (13), mit welcher der Ventilsitz (6) verschließbar ist und die abgetrennt von der Ventilkammer (2) eine Steuerkammer (4) ausbildet, die einen Gaszuführstutzen (8) und einen Gasabführstutzen (9) aufweist dadurch gekennzeichnet, daß der Gasabführstutzen (9) eine Blende (10) enthält.

2. Steuerbares Membranventil nach Anspruch 1, dadurch gekennzeichnet, daß das Öffnungsverhältnis Gasabführstutzen-Innendurchmesser (11) : Blenden-Lochdurchmesser (12) 2 : 1 beträgt.

## Claims

1. Controllable membrane valve for respirators with a gas supply line (5), which in the valve housing (1) leads into a substantially cylindrical valve chamber (2) and terminates there in an annular valve seat (6), as well as with a gas outlet line (7) leading out of the valve chamber (2) and a membrane (13) covering the valve chamber (2) with which the valve seat (6) is able to be sealed and which forms a control chamber (4) separated from the valve chamber (2), which has a gas supply connection (8) and a gas discharge connection (9), characterised in that the gas discharge connection (9) has a shutter (10).

2. Controllable membrane valve according to claim 1, characterised in that the aperture ratio gas discharge connection-inner diameter (11) : shutter aperture diameter (12) is 2 : 1.

## Revendications

1. Soupape à membrane réglable pour respirateurs, avec une conduite d'amenée de gaz (3) qui mène dans le corps de soupape (1) dans une chambre de soupape (2) essentiellement cylindrique, et s'y termine en un siège de soupape (6) de forme annulaire, ainsi qu'avec une conduite de sortie de gaz (7) menant hors de la chambre de soupape (2), et une membrane (13) tendue à travers la chambre de soupape (2) qui permet de fermer le siège de soupape (6) et qui forme une chambre de commande (4), séparée de la chambre de soupape (2), et qui présente un raccord d'amenée de gaz (8) et un raccord d'évacuation de gaz (9), caractérisée en ce que le raccord d'évacuation de gaz (9) contient un diaphragme (10).

2. Soupape à membrane réglable selon la revendication 1, caractérisée en ce que le rapport d'ouverture entre le diamètre intérieur (11) du raccord d'évacuation de gaz et le diamètre (12) de l'orifice du diaphragme est de 2 : 1.